# FASCICULE DE BREVET EUROPEEN

(11) **EP 2 872 027 B1**
(45) Date de publication et mention de la délivrance du brevet: **11.11.2020**
(21) Numéro de dépôt: 13747336.9
(22) Date de dépôt: 10.07.2013
(51) Int. Cl.: A61B 1/04, H01L 27/146

(54) **DISPOSITIF COMPORTANT UN ENSEMBLE DE DETECTEURS SENSIBLES A UNE RADIATION ELECTROMAGNETIQUE ET ARRANGEMENT D'UN ENSEMBLE DE TELS DISPOSITIFS**
VORRICHTUNG MIT EINEM SATZ AUS GEGENÜBER ELEKTROMAGNETISCHER STRAHLUNG EMPFINDLICHEN DETEKTOREN UND ANORDNUNG EINES SATZES AUS DERARTIGEN VORRICHTUNGEN
DEVICE COMPRISING A SET OF ELECTROMAGNETIC RADIATION SENSITIVE DETECTORS AND ARRANGEMENT OF A SET OF SUCH DEVICES

(30) Priorité: 10.07.2012 CH 10622012
(43) Date de publication de la demande: 20.05.2015
(73) Titulaire: AWAIBA Consultadoria, Desenvolvimento e Comércio de Componentes Microelectrónicos, Unipessoal, Lda., Madeira (PT)
(72) Inventeur: WÄNY, Martin, 5656 AE Eindhoven (NL)
(74) Mandataire: Epping - Hermann - Fischer
(86) Numéro de dépôt international: PCT/CH2013/000123
(87) Numéro de publication internationale: WO 2014/008615

(56) Documents cités:
- EP-A1- 1 255 401
- FR-A1- 2 930 841
- JP-A- 2010 273 757
- US-A- 5 291 010
- US-A1- 2006 237 625
- US-A1- 2009 033 777

## Description

### Domaine technique

La présente invention concerne un dispositif comportant un ensemble de détecteurs sensibles à une radiation électromagnétique, notamment un capteur d'images en particulier pour des applications endoscopiques ou des caméras de surveillance miniaturisées, ce capteur comportant une matrice de points d'image agencée pour fournir une image d'une zone explorée dont la forme correspond sensiblement à la géométrie de ladite matrice, dans lequel les détecteurs sont adressés à travers un schéma d'adressage le long des lignes et des colonnes de ladite matrice pour permettre à chaque cellule de ladite matrice de cellules d'être liée à au moins un circuit de lecture placé à la périphérie de ladite matrice, ledit dispositif étant constitué d'une matrice de cellules photodétectrices structurée selon des lignes et des colonnes orthogonales entre elles, qui a une forme polygonale dont le contour comporte au moins cinq côtés inscrits dans une ligne fermée, avec des bords orthogonaux et au moins un bord oblique reliant deux bords orthogonaux, notamment une surface carrée ou rectangulaire avec des angles coupés, en particulier à 45°, dont chaque cellule photodétectrice est directement connectée à un circuit de lecture en tension ou en courant par un système d'adressage le long des lignes et des colonnes.

Elle concerne également un arrangement comportant un ensemble composé de plusieurs de ces dispositifs.

### Technique antérieure

Normalement un dispositif de ce type est constitué d'une matrice de points image, appelés pixels, de forme rectangulaire et le circuit intégré du capteur est également de forme rectangulaire. Néanmoins pour de nombreuses applications endoscopiques et pour d'autres applications où une taille de capteur très petite est exigée, cette forme rectangulaire n'est pas idéale, car la forme rectangulaire ne remplit qu'une partie de la surface circulaire qui correspond habituellement à l'espace disponible pour le dispositif. Le champ de vision couvert par une lentille qui est typiquement circulaire ne correspond pas à la surface sensible du capteur rectangulaire et, soit ne recouvre qu'une partie du champ de vision de la lentille, soit on dispose d'un capteur dont la surface dépasse au niveau de ses angles, le champ de vision couvert par la lentille circulaire.

Vu que la forme rectangulaire pour l'assemblage d'un ensemble de cellules photosensibles afin d'obtenir une image est souvent peu adaptée aux contraintes d'espace à disposition, de nombreuses solutions ont été proposées pour réaliser des capteurs mieux adaptés. Par exemple dans le brevet américain US7009645 on propose un capteur d'image avec un arrangement circulaire des cellules photosensibles. Les cellules photosensibles sont adressées, pour la lecture séquentielle selon un système d'adressage en coordonnées polaires, le long des rayons et en cercles. Cet arrangement a deux défaux majeurs, qui ont mené à ce qu'un tel arrangement est peu utilisé. Premièrement la résolution spatiale d'un tel arrangement de cellules photodétectrices n'est pas uniforme, et augmente vers le centre et, bien que des mesures pour réduire l'impact de ceci soient proposées, une résolution parfaitement uniforme n'est pas obtenue. Deuxièmement, la majorité des algorithmes de traitement d'images et d'affichage d'images sont basés sur un arrangement des points d'image selon des lignes et des colonnes, et donc une conversion à ce format de présentation d'image est impérative pour un tel capteur, ce qui requiert des calculs de transformation d'espace coordonnées très lourds.

On connaît un capteur d'image qui comporte une matrice d'éléments photosensibles dont les signaux individuels sont acheminés selon un processus séquentiel par adressage des lignes et des colonnes desdits éléments photosensibles. Cet adressage séquentiel est réalisé par un circuit d'adressage de ligne placé le long d'un bord périphérique de la matrice et un circuit d'adressage de colonne qui est placé le long d'un autre bord périphérique de la matrice.

Un tel dispositif convient bien lorsque le capteur a une forme carrée ou rectangulaire de sorte que l'adressage des lignes et des colonnes permet de définir les points image sans aucun recoupement. Pour des formes polygonales par exemple, un adressage simple où chaque point d'image a une adresse ligne et une adresse colonne uniques, n'est pas envisageable avec seulement un des éléments soit d'adressage de ligne soit d'adressage de colonnes, placé le long d'un bord périphérique du dispositif dès que le polygone ne contient plus au minimum un angle rectangulaire, car alors au moins le long d'un côté du polygone le décodeur des lignes et le décodeur des colonnes devront être placés en même temps.

Des solutions avec des arrangements de matrices de cellules photodétectrices dont un ou deux coins sont découpés, par exemple comme le montre le brevet US 5291010 ont été proposées mais les circuits d'adressage de lignes et les circuits d'adressage des colonnes restent respectivement sur des côtés individuels du polygone.

Surtout dans le domaine des capteurs de type CCD dans les applications de détection de radiations rayon-X intra orales, des solutions à ce problème ont été proposées, qui peuvent consister à réaliser des arrangements de matrices de cellules photosensibles organisées le long des lignes et des colonnes, mais avec tout les quatre coins coupés (sensiblement de forme octogonales) ont été proposées. Quelques unes de ces solutions ne marchent qu'avec des dispositifs détectrices de type CCD (Dispositif de charge couplée) où la période de la matrice détectrice est sensiblement plus grande que l'espace minimal nécessaire pour l'emplacement de l'électronique de l'adressage, comme c'est souvent le cas dans les applications de Rayon-X intra orales. Dans la technologie CCD on n'a pas besoin d'un adressage le long des lignes proprement dit. Au lieu d'un système d'adressage le long des colonnes, le signal sortant d'une colonne est plutôt transféré le long d'un registre de lecture dit « horizontal », d'une colonne à l'autre. Ainsi la publication européenne EP 1255401 propose une solution pour l'adressage d'une matrice de type CCD destinée à des applications intra-orales avec des coins coupés où l'adressage des lignes passe à travers le registre de lecture horizontal dans une couche conductrice supérieure. Une telle réalisation reste limitée à des matrices de détecteurs de type CCD et ne peut pas être généralisée à des capteurs où chaque pixel doit, pour la lecture, être connecté directement à un circuit de lecture, comme c'est par exemple le cas des matrices de détecteurs réalisées en technologie CMOS (Complementary Metal Oxyde Semiconductor) qui permettent la réalisation des cellules de détecteurs très petites, de l'ordre de quelques micromètres, voir même moins qu'un micromètre pour les pixels les plus petits connu actuellement.

Le brevet américain US2006027625 propose une autre solution réalisable uniquement en technologie CCD pour la réalisation d'une matrice orthogonale avec des coins coupés en oblique, mais qui nécessite un registre de lecture le long des lignes qui peut transférer les charges reçues des colonnes coupées en oblique à chaque coup d'horloge desdits registres de colonnes. Cette propriété est uniquement à disposition dans des détecteurs de type CCD.

Le brevet US20090033777 propose une solution pour une matrice de cellules photodétectrices adressées selon des lignes et des colonnes, qui peut être réalisée en technologie CMOS, mais elle se limite au cas où les côtés obliques sont sensiblement plus petits que les côtés orthogonaux, car les blocs d'adressages, soit des lignes, soit des colonnes des côtés obliques, sont placés derrière le bloc respectif des côtés orthogonaux et sont interconnectés par un réseau de connexions aux côtés obliques. Ce réseau d'interconnexions, va par ailleurs dans un contexte d'application fortement miniaturisée, comme nécessaire dans l'endoscopie par exemple, mener à un surface totale accrue ce qui n'est pas souhaitable.

La publication japonaise JP 210273757 propose une réalisation d'un dispositif comprenant une matrice de cellules photodétectrices de forme généralement circulaire. Néanmoins il est proposé de maintenir la matrice de cellules photodétectrices de forme rectangulaire, voir carrée, mais d'utiliser l'espace à disposition le long des centres des bords orthogonaux de la matrice pour placer d'autres composants électroniques nécessaires à l'opération d'un système de détection de radiation, notamment de capture d'image fortement intégré. Dans une autre variation de cette proposition, il est suggéré de résoudre le problème d'adressage et de lecture de la matrice en plaçant un deuxième élément électronique de lecture dans un deuxième plan de circuit intégrée et d'interconnecter la matrice des cellules photodétectrices par intégration en 3D avec le circuit de lectures. Ceci engendre des coûts de productions par unité importants. Le processus de placement en plaquette de production d'un tel dispositif est limité à la forme hexagonale, et génère, si la plaquette est découpée avec un processus de sciage rectiligne, une perte de plus que 50% de la surface potentielle d'une plaquette de production, de sorte que le coût de production d'un tel dispositif va fortement augmenter. Un processus de sciage alternatif pourrait consister en une coupe circulaire, ce qui n'est pas un procédé standard de la production d'électronique à grande échelle.

La publication française N° 2 930 841 décrit un dispositif comportant un ensemble de détecteurs sensibles à des radiations électromagnétiques pour des applications endoscopiques ayant une configuration similaire à celles du dispositif décrit. Néanmoins la disposition et la géométrie des circuits d'adressage est différente et ne permet pas d'obtenir les mêmes avantages. Plus particulièrement le dispositif décrit dans cette publication impose une augmentation du nombre des transistors intégrés dans chaque pixel, ce qui n'est pas souhaité dans le contexte d'une application endoscopique où la taille de chaque pixel est avantageusement maintenue aussi petite que possible afin d'augmenter la résolution avec un détecteur de surface réduite.

### Exposé de l'invention

La présente invention se propose de fournir une architecture permettant de réaliser des dispositifs de détecteurs sensibles à des radiations électromagnétiques, notamment des capteurs d'image, avec une matrice qui couvre de façon optimale la quasi-totalité de la surface circulaire correspondant au champ visuel de la lentille et une disposition de capteurs adaptée à cette géométrie, cette disposition de capteur étant associée à un adressage qui permet d'identifier sans ambiguïté les points d'image en leur attribuant à chacun une adresse ligne et une adresse colonne uniques. Ils peuvent donc être lus de manière séquentielle classique. Néanmoins l'architecture propose des moyens pour placer les éléments de décodeurs de lignes et adresses le long des bords obliques par rapport aux directions rectilignes des lignes et colonnes d'un schéma d'adressage conventionnel, sans limitation du nombre des coins coupés de ladite matrice.

Par ailleurs la présente invention peut se généraliser à tous les principes de matrices de détecteurs photosensibles, qui requièrent pour la lecture une connexion directe à un ou plusieurs circuits de lecture, notamment à des capteurs d'image réalisés en technologies CMOS. Les principes proposés dans l'invention sont compatibles avec la miniaturisation de la taille des cellules de détection de radiations, peuvent être appliqués à des matrices avec une période aussi faible que 1 à 2 micromètres. En proposant une solution unique pour le placement et le routage des circuits d'adressage de lignes et de colonnes le long des bords obliques de ladite matrice sans augmenter l'espace additionnel au-delà de la matrice de cellules détectrices nécessaire aux bords obliques dudit dispositif pour l'adressage.

Dans ce but, le capteur selon l'invention tel que défini en préambule est caractérisé en ce que les circuits d'adressage des lignes ou des colonnes sont alternativement intercalés par rapport aux circuits d'adressage des colonnes ou des lignes le long dudit au moins un bord oblique, ces circuits d'adressage étant disposés parallèlement entre eux et sensiblement perpendiculairement audit au moins un bord oblique, et en ce que la largeur d'un couple de circuits d'adressage constitué d'une ligne et une colonne aboutissant à une cellule photodétectrice coupée en oblique par ledit bord oblique, est égale à la longueur de la diagonale de ladite cellule photodétectrice coupée en oblique par ce bord oblique.

Selon un mode de réalisation avantageux, dans lequel lesdites cellules photodétectrices sont carrées et ledit au moins un bord oblique est coupé à 45°, la largeur d'un desdits circuits d'adressage peut être sensiblement égal √2/2 multiplié par la dimension d'un côté d'une cellule photodétectrice. La longueur des circuits est dans ce cas sensiblement égale à la longueur des circuits correspondant à la longueur des côtés droits multipliée par √2.

Selon un autre mode de réalisation avantageux, dans lequel lesdites cellules photo détectrices sont carrées et ledit au moins un bord oblique est coupé à 45°, la largeur d'un desdits circuits d'adressage est sensiblement égal √2 multiplié par la dimension d'un côté d'une cellule photodétectrice. La longueur des circuits est dans ce cas sensiblement égale à la longueur des circuits correspondant à la longueur des côtés droits multipliée par √2/2.

Selon un mode de réalisation particulière ladite ligne fermée a une forme circulaire ou une forme ovale.

De façon avantageuse, selon un mode de construction particulier, la forme polygonale de la matrice de cellules photodétectrices est celle d'un octogone inscrit dans un cercle ou une ellipse

Les éléments d'adressage des lignes et des colonnes orthogonales correspondant à la matrice de points d'images dudit capteur peuvent comporter des moyens d'adressage des lignes et des colonnes, ces moyens comportant des éléments disposés le long d'au moins une partie des côtés du contour polygonal de ladite matrice.

Selon un mode de réalisation particulier, les éléments desdits moyens d'adressage des lignes et des colonnes sont alternés le long d'au moins un bord oblique de la matrice de points d'images dudit capteur.

Selon un autre mode de réalisation particulier, les éléments desdits moyens d'adressage des lignes et des colonnes sont disposés les uns derrière les autres le long d'au moins un bord oblique de la matrice de points d'images dudit capteur.

Selon encore un autre mode de réalisation particulier, les éléments desdits moyens d'adressage des lignes et des colonnes disposés le long d'au moins un bord oblique de la matrice de points d'images ont des largeurs réduites par rapport à celle des éléments disposés le long d'un bord orthogonal de la matrice de points d'images dudit capteur.

Selon encore un autre mode de réalisation particulier, les éléments des dits moyens d'adressage des lignes et des colonnes sont disposés le long de tous les bords de la matrice en divisant l'adressage de lignes et de colonnes entre les bords qui s'opposent à travers la matrice.

Le capteur d'images peut être avantageusement monté sur un tube d'endoscope de section sensiblement circulaire et associé à un système optique comportant au moins une lentille circulaire.

L'arrangement selon l'invention est caractérisé en ce que ledit ensemble de dispositifs est réalisé sur une plaquette de production où lesdits dispositifs ont une forme octogonale et où ils sont placés de façon espacée de sorte qu'ils peuvent être découpés avec des lignes droites uniquement qui ont entre eux des angles de 45 degrés uniquement.

### Brève description des figures.

La présente invention et ses avantages seront mieux compris à la lecture de la description détaillée de formes de réalisation préférées du dispositif, en référence aux dessins annexés donnés à titre indicatif et non limitatif, dans lesquels :
La figure 1 représente une vue en plan illustrant une matrice de cellules photodétectrices de l'art antérieur,
La figure 2 représente une vue en perspective de la matrice de cellules photodétectrices de la figure 1 disposée sur un support de section circulaire,
La figure 3 représente une vue qui illustre le problème rencontré lors de l'utilisation d'une matrice de cellules photodétectrices de l'art antérieur et une lentille circulaire,
La figure 4 illustre le mode d'adressage associé à une matrice de cellules photodétectrices de l'art antérieur,
La figure 5 illustre une forme de réalisation d'une matrice de cellules photodétectrices selon l'invention associée à un dispositif d'adressage spécifique,
La figure 6 est une vue partielle qui illustre une forme de réalisation d'un mode d'adressage spécifique adapté à la matrice de cellules photodétectrices de la figure 5,
Les figures 7A et 7B sont des vues partielles qui illustrent d'autres modes d'adressages spécifiques adaptés à la matrice de cellules photodétectrices de la figure 5, et,
la figure 8 est une vue schématique qui illustre un mode de fabrication industrielle d'une série de matrices de cellules photodétectrices pour la réalisation de capteurs selon l'invention.

### Meilleure manière de réaliser l'invention

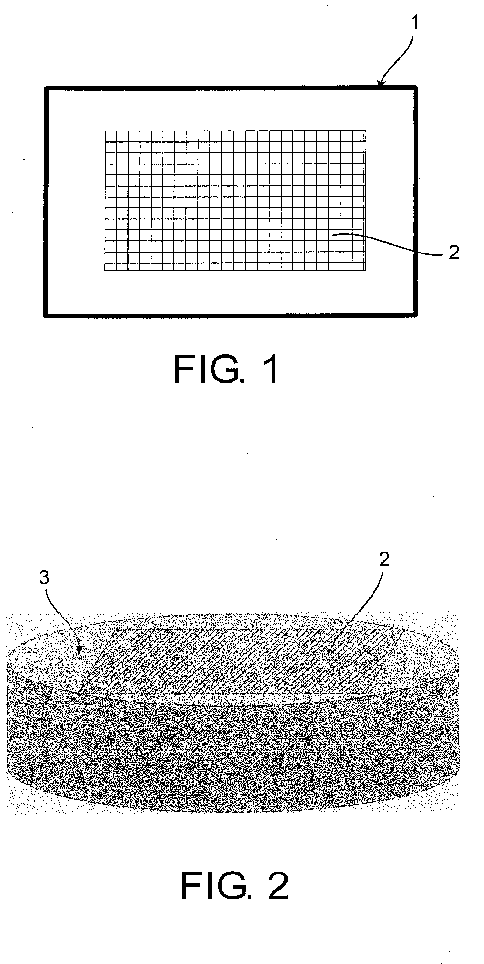

En référence à la figure 1, un capteur de cellules photodétectrices 1 de l'art antérieur se présente habituellement sous une forme rectangulaire ou carrée et les cellules forment une matrice 2 de points d'image ayant également une forme rectangulaire ou carrée. Comme le montre la figure 2, ce capteur est généralement monté sur un support 3 qui peut être un tube d'endoscope de forme circulaire et le système optique associé au capteur, qui comporte une ou plusieurs lentilles est typiquement de section circulaire.

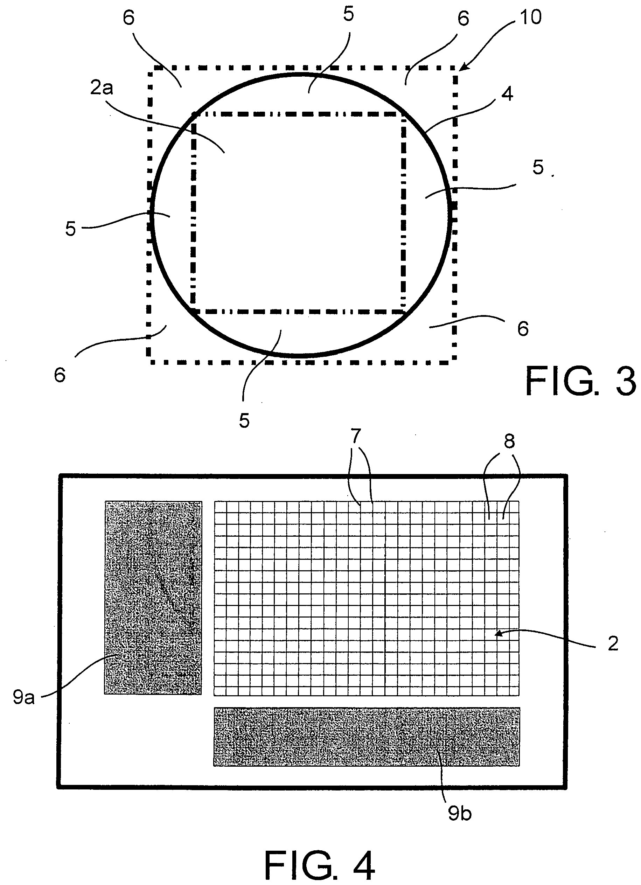

La vue de la figure 3 illustre les surfaces respectives couvertes par une matrice 10 de cellules photodétectrices de l'art antérieur qui a par exemple une forme carrée et de la lentille 4 du système optique, de forme circulaire. Selon une première réalisation la matrice 2a est inscrite dans le cercle représentant la lentille 4. Dans ce cas, les zones 5 qui sont extérieures à la matrice 2a et intérieures au contour circulaire de la lentille 4 ne sont pas couvertes par cette lentille et ne peuvent pas fournir d'image. Selon une deuxième réalisation le cercle représentant la lentille 4 est inscrit dans la matrice 2b. Dans ce cas, les zones 6 qui sont intérieures à la matrice 2a et extérieures au contour circulaire de la lentille 4 ne sont pas couvertes par cette lentille et ne peuvent pas fournir d'image. On aboutit ainsi à une situation qui n'est pas optimale et que la présente invention se propose de corriger.

La figure 4 illustre le mode d'adressage d'un capteur classique de l'art antérieur ayant une matrice de points d'image disposés selon des lignes 7 et des colonnes 8 orthogonales par rapport à ces lignes. Les moyens d'adressage comportent deux éléments 9a et 9b respectivement affectés aux lignes 7 et aux colonnes 8 de la matrice 2 de cellules photodétectrices.

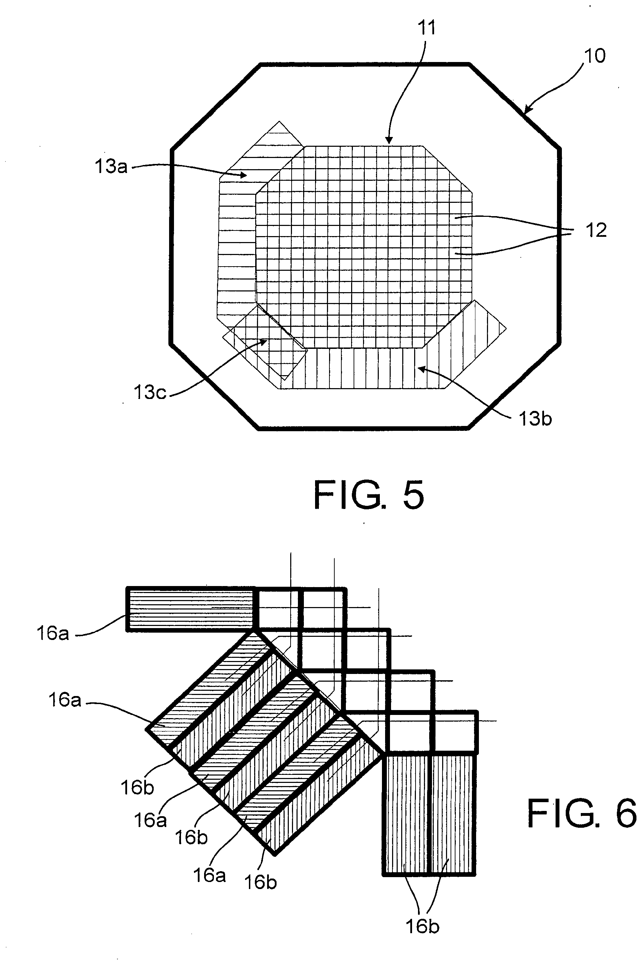

La figure 5 illustre un capteur 10 selon l'invention qui comporte une matrice 11 de cellules photodétectrices 12, cette matrice 11 ayant une forme octogonale. Pour assurer un adressage complet des points d'image de la matrice 11 de cellules photodétectrices 12, il est nécessaire de placer sur au moins un des côtés obliques de la matrice un circuit d'adressage de ligne 13a ainsi qu'un circuit d'adressage de colonne 13b, d'un même côté de la matrice. De cette manière l'extinction de ce circuit d'adressage au-delà de la matrice n'augmente pas significativement de sorte que le bénéfice obtenu par l'utilisation d'une matrice de points d'image dont le contour est polygonal, par exemple octogonal est conséquent et permet une réduction globale de la surface du capteur. Le résultat de cette disposition permet de profiter du fait que la périphérie d'une cellule de la matrice de points d'image coupée non orthogonalement augmente selon les lois de Pythagore, tandis que la dimension de la cellule unitaire du décodeur de lignes, respectivement du décodeur de colonnes n'augmente pas si elle subit une rotation.

Cette augmentation de la périphérie d'une cellule unitaire sur les côtés obliques de la matrice de cellules photodétectrices est utilisée pour placer un élément de base des moyens d'adressage des lignes et des colonnes. La figure 6 illustre les composants du circuit intégré des circuits d'adressage des lignes 16a et des circuits d'adressage des colonnes 16b qui sont disposés côte à côte sur le côté oblique d'une matrice ayant un côté oblique à 45° par rapport aux lignes et aux colonnes. Dans ce cas la largeur d'un couple desdits circuits d'adressage est sensiblement égal à la dimension d'un côté d'une cellule photodétectrice multipliée par √2, soit environ 1,4. Cette augmentation de dimension est utilisée pour placer de façon intercalée les circuits d'adressage de lignes et de colonnes.

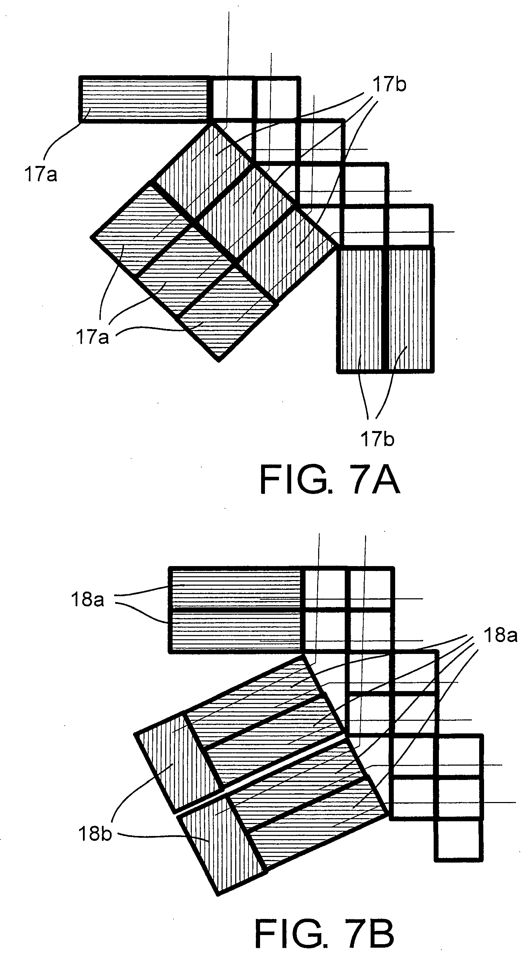

D'autres configurations sont illustrées par les figures 7A et 7B. Dans le cas de la configuration de la figure 7a, l'extinction au-delà de la matrice de lignes et de colonnes peut être réduit en raison de la largeur plus importante des éléments de base des moyens d'adressage de lignes 17a disposés derrière les éléments de base des moyens d'adressage de colonnes 17b. L'inverse serait également réalisable.

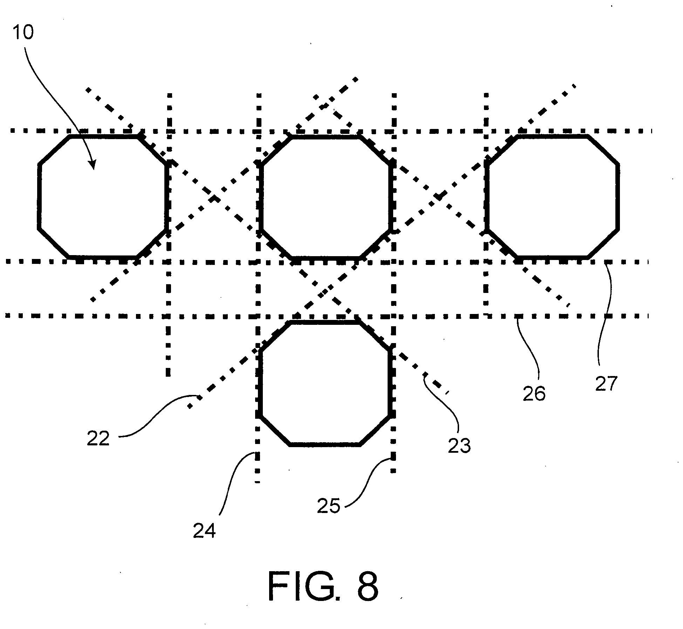

Ce concept peut être étendu à des contours polygonaux de matrices ayant des angles de côtés différents de 45°. La figure 8 décrit une telle configuration où un nombre adéquat d'éléments de base des moyens d'adressage de lignes et de colonnes sont placés dans chaque section de la périphérie de la matrice de points d'image. Le côté oblique de la matrice de la figure 8 a un angle de 26°. On intercale deux éléments de base des moyens d'adressage de lignes 18a entre un élément de base des moyens d'adressage de colonnes 18b.

Selon une variante du capteur selon l'invention les circuits d'adressage des lignes et des colonnes sont distribués le long de tous les côtés de la matrice de sorte que certains groupes de lignes de points images sont adressés d'un côté, tandis que certains autres groupes de lignes de points d'images son adressés du côté opposé, et de même avec les colonnes. Par exemple les lignes paires peuvent être adressées du côté le plus à gauche de la matrice des points d'image, tandis que les lignes paires peuvent être adressées du côté le plus à droite de la matrice des points d'image. Il peut en être de même avec les colonnes paires et impaires des bords inférieurs ou supérieurs de la matrice des points d'image qui peuvent être choisis pour positionner les circuits d'adressage respectifs.

Ainsi en exploitant les principes décrits ci-dessus, il est possible de réaliser un capteur d'image comportant une matrice de forme polygonale, par exemple de forme octogonale, voire décagonale pour se rapprocher de la forme circulaire en augmentant le nombre de côtés, mais également le capteur d'image complet avec les circuits électroniques couvrant une surface polygonale.

Lors de la production à l'échelle industrielle d'un circuit électronique, notamment d'un capteur d'image avec une forme non rectangulaire, notamment polygonale, par exemple octogonale, se pose la question de la séparation des circuits individuels des plaquettes sur lesquelles une pluralité de tels circuits est fabriquée, sachant que les procèdes de séparations de circuits électroniques sont basées sur le principe de la coupure ou du sciage en ligne droite.
A titre d'exemple, la figure 8 illustre la réalisation de plaquettes polygonales initialement selon un arrangement comportant une pluralité de capteurs d'images 20 de forme octogonale sur une plaquette de production 21 de sorte qu'ils puissent être découpés selon des lignes de coupe 22, 23 croisées à 90°, des lignes de coupe parallèles 24, 25 et 26, 27. Il suffit à cet effet de placer les circuits de forme octogonale 19 en lignes et en colonnes espacées entre elles. La découpe peut être faite de telle manière que le côté représenté en haut et à gauche 19a d'un circuit représenté par la figure, correspond au côté en bas à droite 19b d'un autre circuit.

La présente invention n'est pas limitée aux formes de réalisation décrites, mais peut subir différentes modifications ou variantes évidentes pour l'homme du métier. En particulier le nombre des côtés n'est limité que par la réalisation pratique. La forme est adaptable et les moyens d'adressage sont élaborés en fonction de la forme géométrique des matrices d'images.

## Revendications

1. Dispositif comportant un ensemble (10) de détecteurs sensibles à une radiation électromagnétique, notamment un capteur d'images en particulier pour des applications endoscopiques ou des caméras de surveillance miniaturisées, ce capteur comportant une matrice (11) de points d'image agencée pour fournir une image d'une zone explorée dont la forme correspond sensiblement à la géométrie de ladite matrice, dans lequel les détecteurs (12) sont adressés à travers un schéma d'adressage le long des lignes et des colonnes de ladite matrice pour permettre à chaque cellule de ladite matrice de cellules d'être liée à au moins un circuit de lecture placé à la périphérie de ladite matrice, ledit dispositif étant constitué d'une matrice (11) de cellules photodétectrices (12) structurée selon des lignes et des colonnes orthogonales entre elles, qui a une forme polygonale dont le contour comporte au moins cinq côtés inscrits dans une ligne fermée, avec des bords orthogonaux et au moins un bord oblique reliant deux bords orthogonaux, notamment une surface carrée ou rectangulaire avec au moins un angle coupé, en particulier à 45°, définissant des cellules photodétectrices tronquées le long dudit bord oblique, chacune desdites cellules photodétectrices étant directement connectée à un circuit de lecture en tension ou en courant par un système d'adressage le long des lignes (16a) et des colonnes (16b), **caractérisé en que** les circuits d'adressage des lignes ou des colonnes sont placés le long d'au moins un même côté oblique de la matrice de telle sorte que lesdits circuits d'adressage soient disposés parallèlement les uns aux autres et perpendiculairement audit au moins un bord oblique.

2. Dispositif selon la revendication 1, dans lequel lesdites cellules photodétectrices sont carrées et ledit au moins un bord oblique est coupé à 45°, dans lequel lesdites cellules photodétectrices sont carrées et ledit au moins un bord oblique est coupé à 45°, **caractérisé en ce que** la largeur d'un desdits circuits d'adressage est sensiblement égal √2/2 multiplié par la dimension d'un côté d'une cellule photodétectrice, leur longueur étant dans ce cas sensiblement égale à la longueur des circuits correspondant à la longueur des côtés droits multipliée par √2.

3. Dispositif selon la revendication 1, dans lequel lesdites cellules photo détectrices sont carrées et ledit au moins un bord oblique est coupé à 45°, **caractérisé en ce que** la largeur d'un desdits circuits d'adressage est sensiblement égal √2 multiplié par la dimension d'un côté d'une cellule photodétectrice et la longueur des circuits est sensiblement égale à la longueur des circuits correspondant à la longueur des côtés droits multipliée par √2/2.

4. Dispositif selon la revendication 1, **caractérisé en ce que** ladite ligne fermée a une forme circulaire.

5. Dispositif selon la revendication 1, **caractérisé en ce que** ladite ligne fermée a une forme ovale.

6. Dispositif selon la revendication 1, **caractérisé en ce que** les éléments d'adressage des lignes et des colonnes orthogonales correspondant à la matrice de points d'images dudit capteur comportent des moyens d'adressage des lignes et des colonnes, ces moyens comportant des éléments disposés le long d'au moins une partie des côtés du contour polygonal de ladite matrice.

7. Dispositif selon la revendication 6, **caractérisé en ce que** les éléments desdits moyens d'adressage des lignes et des colonnes disposés le long d'au moins un bord oblique de la matrice de points d'images ont des largeurs réduites par rapport à celle des éléments disposés le long d'un bord orthogonal de la matrice de points d'images dudit capteur.

8. Dispositif selon la revendication 6, **caractérisé en ce que** les éléments desdits moyens d'adressage des lignes et des colonnes sont disposés le long de tous les bords de la matrice en divisant l'adressage de lignes et de colonnes entre les bords qui s'opposent au travers de la matrice.

9. Dispositif selon la revendication 6, **caractérisé en ce qu'**il est monté sur un tube d'endoscope de section sensiblement circulaire et associé à un système optique comportant au moins une lentille circulaire.

10. Dispositif selon l'une quelconque des revendications précédentes, **caractérisé en ce qu'**il est réalisé en technologie CMOS (Complementary Metal Oxyde Semiconductor)

11. Arrangement de fabrication d'un ensemble de dispositifs selon l'une quelconque des revendications précédentes, **caractérisé en ce que** ledit ensemble de dispositifs est réalisé sur une plaquette de production où lesdits dispositifs ont une forme octogonale et où ils sont placés de façon espacée de sorte qu'ils peuvent être découpés avec des lignes droites uniquement qui ont entre eux des angles de 45 degrés uniquement.

## Patentansprüche

1. Vorrichtung, umfassend eine Einheit (10) von für elektromagnetische Strahlung empfindlichen Detektoren, insbesondere einen Bildsensor, insbesondere für endoskopische Anwendungen, oder miniaturisierte Überwachungskameras, wobei dieser Sensor eine Matrix (11) von Bildpunkten umfasst, die dazu eingerichtet sind, ein Bild eines untersuchten Bereichs zu liefern, dessen Form im Wesentlichen der Geometrie der Matrix entspricht, wobei die Detektoren (12) über ein Adressierungsschema entlang der Zeilen und Spalten der Matrix angesprochen werden, um jede Zelle der Zellenmatrix mit mindestens einer Leseschaltung verbunden sein zu lassen, die am Umfang der Matrix angeordnet ist, wobei die Vorrichtung aus einer entlang der zueinander orthogonalen Zeilen und Spalten strukturierten Matrix (11) von Fotoerfassungszellen (12) besteht, die eine polygonale Form hat, deren Kontur mindestens fünf in einer geschlossenen Linie eingeschriebene Seiten mit orthogonalen Rändern und mindestens einem, zwei orthogonale Ränder verbindenden schrägen Rand umfasst, vor allem eine viereckige oder rechteckige Fläche mit mindestens einer insbesondere auf 45° abgeschnittenen Ecke, welche die entlang des schrägen Rands abgeschnittenen Fotoerfassungszellen definiert, wobei jede der Fotoerfassungszellen über ein Adressierungssystem entlang der Zeilen (16a) und der Spalten (16b) spannungs- oder strommäßig direkt an eine Leseschaltung angeschlossen ist, **dadurch gekennzeichnet, dass** die Adressierungsschaltungen der Zeilen oder der Spalten sich entlang mindestens einer selben schrägen Seite der Matrix befinden, und zwar derart, dass die Adressierungsschaltungen zueinander parallel und zu dem mindestens einen schrägen Rand senkrecht angeordnet sind.

2. Vorrichtung nach Anspruch 1, wobei die Fotoerfassungszellen viereckig sind und der mindestens eine schräge Rand auf 45° abgeschnitten ist, wobei die Fotoerfassungszellen viereckig sind und der mindestens eine schräge Rand auf 45° abgeschnitten ist, **dadurch gekennzeichnet, dass** die Breite einer der Adressierungsschaltungen im Wesentlichen gleich √2/2 multipliziert mit der Abmessung einer Seite einer Fotoerfassungszelle ist, wobei ihre Länge in diesem Fall im Wesentlichen gleich der Länge der Schaltungen entsprechend der Länge der geraden Seiten multipliziert mit √2 ist.

3. Vorrichtung nach Anspruch 1, wobei die Fotoerfassungszellen viereckig sind und der mindestens eine schräge Rand auf 45° abgeschnitten ist, **dadurch gekennzeichnet, dass** die Breite einer der Adressierungsschaltungen im Wesentlichen gleich √2 multipliziert mit der Abmessung einer Seite einer Fotoerfassungszelle ist, und die Länge des Schaltungen im Wesentlichen gleich der Länge des Schaltungen entsprechend der Länge der geraden Seiten multipliziert mit √2/2 ist.

4. Vorrichtung nach Anspruch 1, **dadurch gekennzeichnet, dass** die geschlossene Linie eine Kreisform hat.

5. Vorrichtung nach Anspruch 1, **dadurch gekennzeichnet, dass** die geschlossene Linie eine ovale Form hat.

6. Vorrichtung nach Anspruch 1, **dadurch gekennzeichnet, dass** die Adressierungselemente der orthogonalen Zeilen und Spalten, die der Bildpunktmatrix des Sensors entsprechen, Adressierungsmittel für die Zeilen und Spalten umfassen, wobei diese Mittel Elemente umfassen, die entlang mindestens eines Teils der Seiten der polygonalen Kontur der Matrix angeordnet sind.

7. Vorrichtung nach Anspruch 6, **dadurch gekennzeichnet, dass** die Elemente der Adressierungsmittel für die Zeilen und Spalten, die entlang mindestens einen schrägen Rands der Bildpunktmatrix angeordnet sind, Breiten haben, die gegenüber derjenigen der Elemente reduziert sind, die entlang eines orthogonalen Rands der Bildpunktmatrix des Sensors angeordnet sind.

8. Vorrichtung nach Anspruch 6, **dadurch gekennzeichnet, dass** die Elemente der Adressierungsmittel für die Zeilen und Spalten entlang aller Ränder der Matrix angeordnet sind und dabei die Adressierung von Zeilen und Spalten zwischen den Rändern aufteilen, die sich über die Matrix hinweg gegenüberliegen.

9. Vorrichtung nach Anspruch 6, **dadurch gekennzeichnet, dass** sie an einem Endoskoprohr im Wesentlichen kreisförmigen Querschnitts angebracht und mit einem optischen System verbunden ist, das mindestens eine kreisförmige Linse umfasst.

10. Vorrichtung nach einem der vorhergehenden Ansprüche, **dadurch gekennzeichnet, dass** sie in CMOS-Technologie hergestellt ist.

11. Anordnung zur Fertigung einer Einheit von Vorrichtungen nach einem der vorhergehenden Ansprüche, **dadurch gekennzeichnet, dass** die Einheit von Vorrichtungen auf einem Produktionsplättchen hergestellt wird, wo die Vorrichtungen eine achteckige Form haben und wo sie beabstandet so angeordnet sind, dass sie ausschließlich mit geraden Zeilen abgeschnitten werden können, die untereinander ausschließlich Winkel von 45 Grad haben.

## Claims

1. A device, comprising a unit (10) of detectors that are sensitive to electromagnetic radiation, in particular an image sensor, in particular for endoscopic applications, or miniaturized monitoring cameras, wherein said sensor comprises a matrix (11) of pixels arranged to provide an image of the explored area, the shape of which substantially corresponds to the geometry of the matrix, wherein the detectors (12) are addressed via an addressing scheme along the lines and columns of the matrix, for permitting each line of the cell matrix to be connected to at least one reading circuit arranged at the periphery of the matrix, wherein the device is composed of a matrix (11) of photodetection cells (12) structured along the mutually orthogonal lines and columns, which has a polygonal shape, the contour of which comprises at least five sides inscribed in a closed line with orthogonal edges, and at least one oblique edge connecting two orthogonal edges, in particular a square or rectangular surface with at least one cut corner, in particular cut at 45°, which defines the photodetection cells cut along the oblique edge, wherein each of the photodetection cells is connected directly in terms of tension or current via an addressing system along the lines (16a) and columns (16b) to a reading circuit, **characterized in that** the addressing circuits of the lines or the columns are located along at least one same side of the matrix such that the addressing circuits are arranged mutually parallel and perpendicular to the at least one oblique edge.

2. The device according to claim 1, wherein the photodetection cells are square and the at least one oblique edge is cut at 45°, **characterized in that** the width of one of the addressing circuits is substantially equal to √2/2 multiplied by the dimension of one side of a photodetection cell, wherein its length is in this case substantially equal to the length of the circuits corresponding to the length of the straight sides multiplied by √2.

3. The device according to claim 1, wherein the photodetection cells are square and the at least one oblique edge is cut at 45°, **characterized in that** the width of one of the addressing circuits is substantially equal to √2 multiplied by the dimension of one side of a photodetection cell, and the length of the circuits is substantially equal to the length of the circuits corresponding to the length of the straight sides multiplied by √2/2.

4. The device according to claim 1, **characterized in that** the closed line has a circular shape.

5. The device according to claim 1, **characterized in that** the closed line has an oval shape.

6. The device according to claim 1, **characterized in that** the addressing elements of the orthogonal lines and columns corresponding to the pixel matrix of the sensor, comprise addressing means for the lines and columns, wherein these means comprise elements arranged along at least a part of the sides of the polygonal contour of the matrix.

7. The device according to claim 6, **characterized in that** the elements of the addressing means for the lines and columns arranged along at least an oblique edge of the pixel matrix, have widths which are reduced as compared to that of the elements arranged along an orthogonal edge of the pixel matrix of the sensor.

8. The device according to claim 6, **characterized in that** the elements of the addressing means for the lines and columns are arranged along all of the edges of the matrix while dividing the addressing of lines and columns between the edges opposing across the matrix.

9. The device according to claim 6, **characterized in that** it is mounted on an endoscope tube of a substantially circular cross section and is associated with an optical system comprising at least one circular lens.

10. The device according to any one of the preceding claims, **characterized in that** it is realized in CMOS technology.

11. An arrangement for fabricating a unit of devices according to any one of the preceding claims, **characterized in that** the unit of devices is realized on a production wafer where the devices have an octagonal shape and where they are arranged in a spaced manner such that they can be cut exclusively by straight lines mutually having angles of exclusively 45 degrees.
